# EUROPÄISCHE PATENTSCHRIFT

(11) **EP 0 791 344 B1**
(45) Veröffentlichungstag und Bekanntmachung des Hinweises auf die Patenterteilung: **10.10.2001**
(21) Anmeldenummer: 97102433.6
(22) Anmeldetag: 14.02.1997
(51) Int. Cl.: A61F 2/38

(54) **Kniegelenkendoprothese**
Knee joint endoprosthesis
Endoprothese de l'articulation du genou

(30) Priorität: 21.02.1996 DE 19606461; 07.05.1996 DE 19618321
(43) Veröffentlichungstag der Anmeldung: 27.08.1997
(73) Patentinhaber: PLUS ENDOPROTHETIK AG, 6343 Rotkreuz (CH)
(72) Erfinder: Schmotzer, Hans, Dr., 5000 Aarau (CH); Horber, Willi, Dr., 8005 Zürich (CH)
(74) Vertreter: Popp, Eugen, Dr.

(56) Entgegenhaltungen:
- EP-A- 0 627 203
- EP-A- 0 716 839
- US-A- 4 262 368

## Beschreibung

Die Erfindung betrifft eine Kniegelenkendoprothese, mit einem Femurteil, das am unteren Ende eines Femurschaftes konvex gekrümmte Kondylenschalen mit ersten Gleitflächen aufweist, die dorsal zwei voneinander beabstandete, einen interkondylären Zwischenraum bildende Seitenwände besitzen, mit einem Tibiateil, das am oberen Ende eines Tibiaschaftes ein Tibiaplateau aufweist, auf dem den ersten Gleitflächen der Kondylenschalen entsprechende zweite Gleitflächen ausgebildet sind, und das eine sich axial in dem Tibiaschaft erstreckende Ausnehmung aufweist, und mit einem Kupplungsteil, das am oberen Ende eines drehbar in der Ausnehmung aufgenommenen Kupplungszapfens einen in den interkondylären Zwischenraum hineinragenden Gelenkkopf aufweist, der um eine quer zur Femurachse verlaufende Schwenkachse schwenkbar gelagert ist.

Kniegelenkendoprothesen, die mittels eines Scharniergelenks die Flexions-/Extensionsbewegung zwischen Femur und Tibia ermöglichen, werden allgemein auch als "Scharnierprothesen" bezeichnet, und deren Schwenkachse auch als "Scharnierachse". Eine derartige Scharnierprothese ist beispielsweise aus der DE 35 29 894 C2 oder aus der EP 0 174 531 bekannt. Sie koppeln beim Zusammensetzen der Prothese nicht mehr an der Scharnierachse, sondern an dem Kupplungszapfen an, der entweder an der Tibia befestigt ist und nach oben ragt und mit einem am Scharniergelenk befestigten Ring artikuliert, oder der am Scharniergelenk befestigt ist und in die Ausnehmung des Tibiateils eingesetzt wird. Dabei erfüllt der Kupplungszapfen des Kupplungsteils im wesentlichen drei Aufgaben: zum einen ermöglicht er zusätzlich zur Flexions-/Extensionsbewegung der Scharnierachse eine Rotation um die Tibiaachse. Zum anderen überträgt er die anterior/posterioren Schubkräfte vom Femur auf die Tibia. Schließlich erlaubt der Kupplungszapfen die Distraktion des Kniegelenks bei Zugbeanspruchung, so daß diese nicht auf die Verankerung der Endoprothese übertragen wird.

Die der vorliegenden Erfindung zugrundeliegende Problemstellung hängt mit der dritten, von dem Kupplungszapfen zu erfüllenden Aufgabe, nämlich der Ermöglichung einer Distraktion des Kniegelenks bei Zugbeanspruchung, zusammen. Dabei ergibt sich nämlich das Problem, daß der mögliche Distraktionsweg, also die erforderliche Länge des Kupplungszapfens, groß genug sein muß, um auch bei massiv gespannten Bändern, also einer extremen Distraktion, eine Dislokation der Baugruppen der Gelenkprothese zu vermeiden. Je länger aber der Kupplungszapfen ist, um so schwieriger ist ein schonendes intraoperatives Zusammenkoppeln der Prothesenteile, was in vielen Fällen gar nicht mehr möglich sein wird, es sei denn, die verbliebenen Bandstrukturen des Kniegelenks werden mit Gewalt gedehnt, gezerrt oder mit dem Messer abgelöst.

An dieser Problemstellung setzt die vorliegende Erfindung an, als deren Aufgabe es angesehen wurde, eine Kniegelenkendoprothese der eingangs genannten Art derart weiterzubilden, daß auch bei längeren Kupplungszapfen ohne weiteres ein schonendes intraoperatives Zusammenkoppeln der Prothesenteile möglich ist.

Diese Aufgabe wird bei der Kniegelenkendoprothese der eingangs genannten Art erfindungsgemäß dadurch gelöst, daß sich der Querschnitt der Ausnehmung des Tibiateils zum Tibiaplateau hin von der Tiefe der Ausnehmung einseitig zu einem Langloch im Tibiaplateau vergrößert.

Somit wird die Öffnungsweite der Ausnehmung zur Aufnahme des Kupplungszapfens vergrößert und gleichzeitig verlagert, so daß ein nach proximal divergierender Schlitz entsteht. Die Vorteile der erfindungsgemäßen Lösung liegen insbesondere darin, daß das Knie beim Zusammenkoppeln der Prothesenteile nicht um die Länge des Kupplungszapfens gedehnt werden muß, sondern dieser unter Ausnutzung des zusätzlichen Freiraums, welcher durch die divergierende Form der Ausnehmung zur Verfügung steht, durch einfaches Schwenken in das Tibiateil eingeführt werden kann. Dadurch ist beim Zusammenkoppeln der Prothesenteile eine wesentlich geringere Dehnung der Bänder erforderlich.

Vorteilhafte Weiterbildungen sind in den Unteransprüchen angegeben. So ist vorzugsweise vorgesehen, daß die Längsachse des Langlochs im wesentlichen von posterior nach anterior verläuft, während der axiale Teil der Ausnehmung im wesentlichen parallel oder deckungsgleich mit der Tibiaachse verläuft. Die Zunahme des Öffnungsquerschnitts der Ausnehmung wird somit nach anterior verlagert, weshalb der Femurteil auf wesentlich erleichterte Art und Weise von posterior in zunächst schräger Haltung eingesetzt werden kann.

Zur genaueren Ausrichtung des divergierenden, den zusätzlichen Freiraum schaffenden Teils der Ausnehmung ist vorgesehen, daß die anteriore, von der Tiefe der Ausnehmung schräg nach oben in Richtung des Tibiaplateaus verlaufende Innenkante der divergierenden Ausnehmung bei zusammengesetzter Prothese eine Tangente an einen Kreisbogen um die Schwenkachse des Gelenkkopfes ist. Hierbei wird der Verlauf der Innenkante im Längsschnitt der Ausnehmung durch den von unten nach oben verlaufenden Scheitelpunkt des Langlochs definiert und der Winkel α zwischen der Tangente und der Tibiaachse ist ein Maß für die Neigung jener Innenkante. Diese kann wahlweise geradlinig, konkav oder konvex gewölbt, oder in Form einer Kombination von mindestens zwei dieser Formen ausgebildet sein. Darüber hinaus kann es sich beim Einsetzen des Femurteils vorteilhaft auswirken, wenn die Seitenwände der divergierenden Ausnehmung wahlweise ebenfalls zum Tibiaplateau hin oder nach anterior oder sowohl nach anterior als auch zum Tibiaplateau hin divergieren. Hierbei führt die Divergenz nach anterior zu einer sich nach anterior öffnenden trapezähnlichen Querschnittsform, wobei die Innenform der Ausnehmung wahlweise seitensymmetrisch oder seitenasymmetrisch ausgebildet sein kann.

Vorzugsweise weist der axiale Teil der Ausnehmung eine größere axiale Erstreckung auf, als der divergierende Teil. Dadurch wird erreicht, daß die Kopplung der Prothesenteile mit einer eindirektionalen Absenkung des Femurteils in das Tibiateil endet.

Für Kniegelenkendoprothesen der hier beschriebenen Art ist es üblich, zwischen der Tragfläche des Tibiaplateaus und den ersten Gleitflächen der Kondylenschalen ein Inlay anzuordnen, was den Verschleiß der Prothese vermindert. Ein solches Inlay weist eine Bodenfläche, zwei Gleitflächen auf der Oberseite und eine Bohrung zum Durchführen des Kupplungszapfens auf. In Weiterbildung der vorliegenden Erfindung ist vorzugsweise vorgesehen, daß an der Bodenfläche des Inlays ein Hülsenkörper angesetzt ist, dessen Hohlraum mit der Bohrung des Inlays fluchtet, und dessen äußere Form im wesentlichen komplementär zur Innenform der Ausnehmung des Tibiateils ausgebildet ist. Die wesentlichen Vorteile eines solchen Hülsenkörpers als Bestandteil des Inlays bestehen insbesondere in der Handhabung der Prothese und dem vereinfachten intraoperativen Zusammenkoppeln ihrer Bauteile: zum Zusammenkoppeln wird nämlich zunächst das Inlay mit dem Hülsenkörper auf den Kupplungszapfen gesteckt und dann wird der Kupplungszapfen zusammen mit dem Inlay in die divergierende Ausnehmung des Tibiateils eingeführt. Indem die axiale Bohrung der Ausnehmung etwas tiefer ausgeführt ist als der divergierende Teil, endet das Zusammenkoppeln mit einer axialen Absenkung des Inlays in das Tibiateil, wobei ein vollendeter Formschluß des Inlays mit dem Seitenbord der Tragfläche des Tibiaplateaus sowie des Hülsenkörpers mit der Ausnehmung des Tibiateils erfolgt. Passende Sicherungsmittel, wie Schrauben, Schnappvorrichtungen oder ähnliches, verhindern eine nachträgliche Dislokation des Zwischenkörpers vom Tibiateil.

Die mit der erfindungsgemäßen Kniegelenkendoprothese durchführbare Reihenfolge des Zusammenkoppelns der Prothesenteile, nämlich zunächst die Verbindung des Inlays mit dem Femurteil bzw. dem daran befestigten Kupplungsteil, und danach die Verbindung jener beiden Teile mit dem Tibiateil, hebt sich in äußerst vorteilhafter Weise gegenüber der Reihenfolge des Zusammenkoppelns bei bekannten Kniegelenkendoprothesen ab, bei denen zuerst das Femurteil mit dem Tibiateil gekoppelt und dann das Inlay eingefügt wird.

Der Hülsenkörper kann einstückig mit der Bodenfläche des Inlays oder als separates Bauteil ausgebildet sein. In der Ausführungsform als separates Bauteil sind drei Varianten vorgesehen, von denen sich die ersten beiden Varianten durch die Art, wie der Hülsenkörper vom Inlay abgetrennt wird, voneinander unterscheiden.

Bei der ersten Variante ist im Inlay eine Aussparung zum Einsetzen des Hülsenkörpers vorzusehen, was beispielsweise durch vier senkrechte Schnitte durch das Inlay erfolgt. In diesem Fall würde beim Zusammenkoppeln zuerst das Inlay mit dem Tibiateil zusammengefügt und dann der Kupplungszapfen mit dem aufgesteckten Hülsenkörper in die divergierende Ausnehmung des Tibiateils eingeführt. Auch hier würden passende Sicherungsmittel eine nachträgliche Dislokation von Hülsenkörper und Inlay verhindern. Bei dieser Ausführungsform könnte das Inlay auch werkstattseitig vormontiert sein, was das Dislokationsrisiko weiter herabsetzen würde.

Bei der ersten Variante als separates Bauteil kann als weitere vorteilhafte Ausgestaltung vorgesehen sein, daß die Oberkante des Hülsenkörpers nach dem Einsetzen in die Aussparung in dem Inlay mit der Oberseite des Inlays fluchtet.

Bei der zweiten Variante des Hülsenkörpers als separates Bauteil ist in dem Inlay anstelle der anterioren Aussparung eine posteriore in Form eines Schlitzes angebracht. Hierzu wird der Hülsenkörper beispielsweise durch einen einzigen horizontalen Schnitt von der Unterseite des Inlays abgetrennt. Beim Zusammenkoppeln würde zuerst der Kupplungszapfen mit dem aufgesteckten Hülsenkörper in die divergierende Ausnehmung des Tibiateils eingeführt und dann das Inlay über den Hülsenkörper hinweg auf das Tibiaplateau geschoben. Hierbei fluchtet dann vorzugsweise die Oberkante des Hülsenkörpers nach dem Einsetzen in die Ausnehmung des Tibiateils mit der Tragfläche des Tibiaplateaus. Der Vorteil dieser Ausführungsform liegt in der vereinfachten Wahl eines passend dicken Inlays. Darüber hinaus sichert das Inlay den Hülsenkörper zusätzlich gegen eine Dislokation. Die posteriore Aussparung im Inlay kann durch einen am Tibiaplateau vormontierten Füllkörper überbrückt oder auch frei gelassen werden. Dies ist möglich, weil dem posterioren Zentralbereich keine wichtige Funktion zukommt.

Die dritte Variante ist eine Untervariante zur ersten und zweiten Variante. Bei ihr ist der Hülsenkörper längs der Tibiaachse geteilt ausgebildet, so daß eine posteriore und eine anteriore Halbschale entstehen. Der posteriore Teil bildet mit dem vorgenannten Füllkörper ein eigenes Bauteil und wird auf dem Tibiateil vormontiert. Der anteriore Teil kann ebenfalls, wie in den obigen Beispielen, als separates Bauteil ausgebildet oder an der Unterseite des Inlays angefügt sein. Das Zusammenkoppeln erfolgt dann entsprechend den vorstehend beschriebenen Ausführungsformen.

Im folgenden wird ein bevorzugtes Ausführungsbeispiel der Erfindung anhand einer Zeichnung näher erläutert.

Es zeigen:
- Fig. 1: eine Explosionsdarstellung einer Kniegelenkendoprothese;
- Fig. 2: einen ventral-dorsalen Vertikalschnitt durch einen Tibiaabschnitt, ein Tibiateil sowie ein Femurteil und ein Inlay beim Einsetzen;
- Fig. 3: eine der Fig. 2 entsprechende Darstellung, allerdings mit eingesetztem Femurteil und Inlay; und
- Fig. 4: eine schematische Draufsicht auf die Tragfläche des Tibiaplateaus.

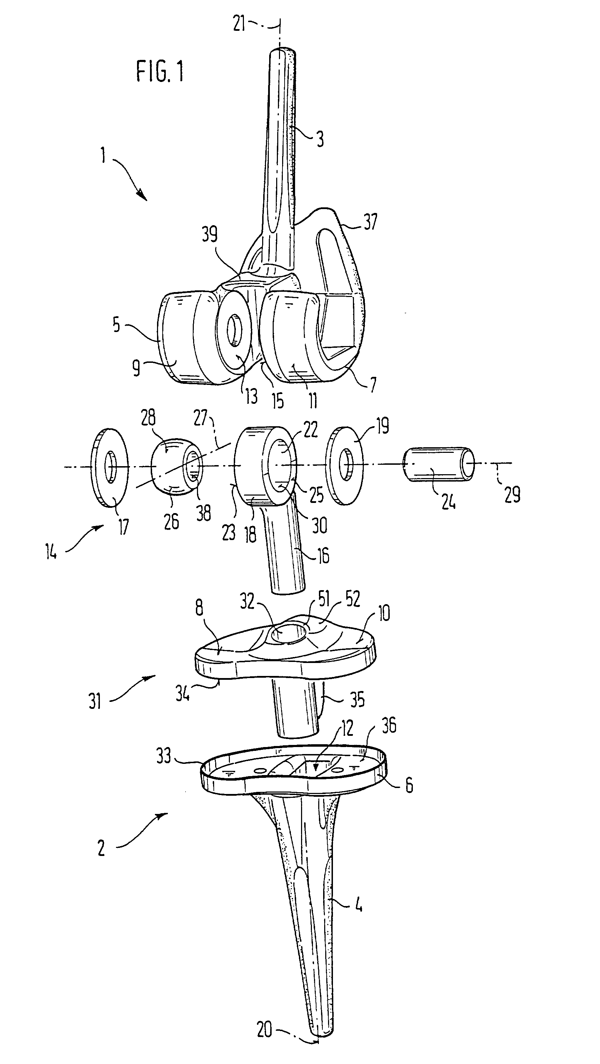

Fig. 1 zeigt eine Explosionsdarstellung einer Kniegelenkendoprothese, die im wesentlichen aus vier Baugruppen besteht: einem Femurteil 1 mit einem Femurschaft 3 und an dessen unterem Ende angeordneten konvex gekrümmten Kondylenschalen 5, 7, welche den natürlichen Kondylen weitgehend angepaßt sind und auf ihrer Unterseite erste Gleitflächen 9, 11 aufweisen, und die dorsal zwei voneinander beabstandete Seitenwände 13, 15 aufweisen, die mit einem Steg zu einem Kasten 39 verbunden sind und innerhalb des Kastens 39 einen interkondylären Zwischenraum bilden. Die beiden Kondylenschalen 5, 7 gehen ventral ineinander über und bilden dort ein Patellaschild 37 zur Artikulation mit der Patella.

Das Pendant zum Femurteil 1 ist ein Tibiateil 2 mit einem Tibiaschaft 4 und einem Tibiaplateau 6 an dessen oberem Ende, welches eine Tragfläche 36 aufweist, die eine sich axial in dem Tibiaschaft 4 erstreckende Ausnehmung 12 sowie ein Seitenbord 33 zur Aufnahme einer dritten Baugruppe 31 aufweist.

Diese dritte Baugruppe 31 besteht aus einem Inlay mit einer Bodenfläche 34 zur Auflage auf der Tragfläche 36 des Tibiateils 2, mit einem an der Bodenfläche 34 ansetzenden Hülsenkörper 35, der zur Befestigung des Inlays 31 an dem Tibiateil 2 in die Ausnehmung 12 des Tibiateils 2 gesteckt wird, ferner mit zweiten Gleitflächen 8, 10 auf der Oberseite des Inlays 31, welche in ventral-dorsaler Richtung konkav gekrümmt sind und auf denen sich die Kondylenschalen 5, 7 des Femurteils 1 abstützen. Zur Befestigung der vierten Baugruppe, dem Kupplungsteil 14, an dem Tibiateil 2 weist das Inlay 31 eine Bohrung 32 auf, welche sich durch die Oberseite des Inlays und durch den Hülsenkörper 35 erstreckt.

Das Kupplungsteil 14 weist als zentrales Element einen Kupplungszapfen 16 und an dessen oberem Ende einen in den interkondylären Zwischenraum des Femurteils 1 hineinragenden Gelenkkopf 18 auf. Im zusammengebauten Zustand der Kniegelenkendoprothese ist der Kupplungszapfen 16 in der Bohrung 32 des Inlays 31 und damit in der Ausnehmung 12 des Tibiateils 2 drehbar aufgenommen. Der Gelenkkopf 18 besitzt eine quer zur Tibiaachse 20 bzw. quer zur Femurachse 21 verlaufende Bohrung 22 und ist mittels eines Kupplungsbolzens 24 um eine im wesentlichen horizontale Gelenkachse 29 schwenkbar in den Seitenwänden 13, 15 der Kondylenschalen 5, 7 gelagert. Die Bohrung 22 in dem Gelenkkopf 18 weist eine Gleitfläche 30 auf, die derart geformt ist, daß sie einen Lagersitz für eine Gleithülse 26 bildet, die im zusammengesetzten Zustand der Kniegelenkendoprothese zwischen dem Kupplungsbolzen 24 und dem Gelenkkopf 18 angeordnet, d.h. mit ihrer Bohrung 38 auf den Kupplungsbolzen 24 aufgesteckt ist. In der in Fig. 1 dargestellten Ausführungsform weist die Gleithülse eine sphärische Außenmantelfläche 28 auf, die mit der entsprechend geformten Gleitfläche 30 des Gelenkkopfes 18 zusammenwirkt und eine Aufteilung der Gelenkflächen auf den zylindrischen Kupplungsbolzen 24 und die sphärische Gleithülse 26 ermöglicht: der zylindrische Kupplungsbolzen 24 übernimmt ausschließlich die Flexions-/Extensionsbewegung zwischen dem Kupplungsbolzen und der Gleithülse, während die sphärische Gleithülse 26 sowohl die Varus-/Valgusbewegung als auch eine Rotationsbewegung zwischen Gleithülse und Gelenkkopf übernimmt. Die Außenmantelfläche 28 könnte selbstverständlich auch irgendeine andere zweckdienliche Form aufweisen. Zur Verschleißminderung zwischen den Seitenflächen 23, 25 des Gelenkkopfes 18 einerseits und den dagegen drehenden Seitenwänden 13, 15 der Kondylenschalen 5, 7 andererseits sind Scheiben 17, 19 vorgesehen, die ebenfalls auf den Kupplungsbolzen 24 aufgesteckt sind.

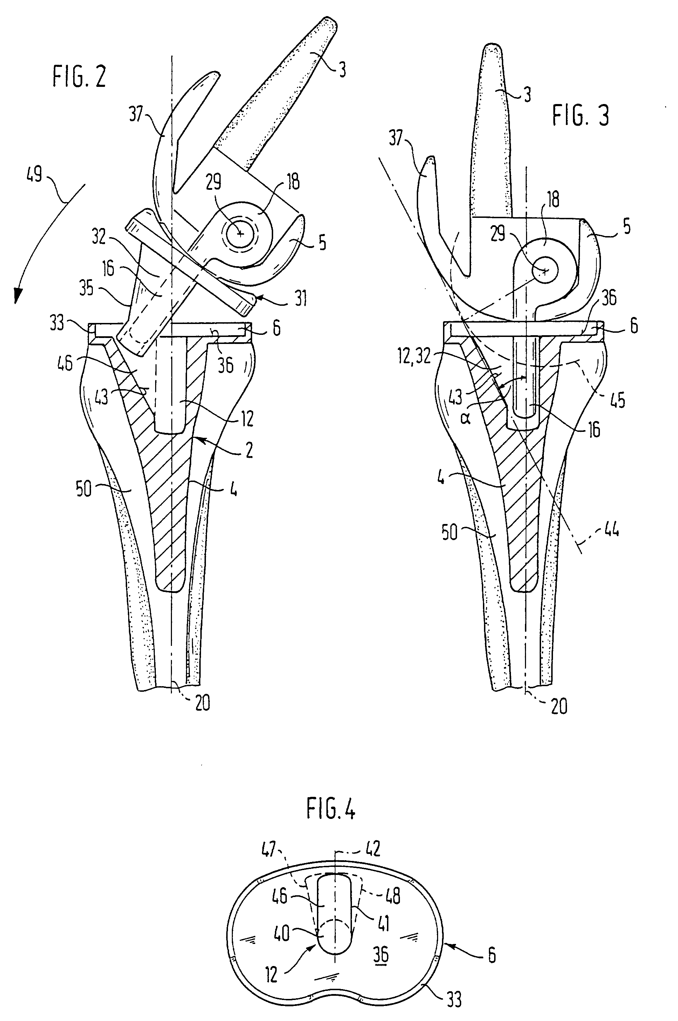

Fig. 2 zeigt einen ventral-dorsalen Vertikalschnitt durch einen oberen Tibiaabschnitt 50, ein in den Tibiaabschnitt 50 eingesetztes Tibiateil 4 sowie ein zum Einsetzen (Zusammenkoppeln) angesetztes Femurteil 3 mit einem Inlay 31. Das Kupplungsteil mit dem Kupplungszapfen 16 und dem Gelenkkopf 18 ist mittels des vorstehend anhand von Fig. 1 beschriebenen Kupplungsbolzens in den interkondylären Zwischenraum eingehängt und um eine Schwenkachse 29 schwenkbar gelagert. Bereits auf den Kupplungszapfen 16 aufgesteckt ist das Inlay 31 mit dem Hülsenkörper 35.

Anhand dieses Vertikalschnitts durch das Tibiateil ist erkennbar, daß die Ausnehmung 12, welche sich mit einem ersten Teil axial in dem Tibiaschaft 4 erstreckt, in einem weiteren Teil 46 von unten nach oben nach anterior (in der Darstellung links) divergent vergrößert. Die anteriore Innenkante 43 der Ausnehmung 12 verläuft somit von der Tiefe der Ausnehmung schräg in Richtung des Tibiaplateaus 6. Der durch diese divergierende Formgebung der Ausnehmung 12 geschaffene zusätzliche Freiraum 46, der im wesentlichen von posterior nach anterior verläuft, ermöglicht selbst bei längeren Kupplungszapfen 16 ein intraoperatives Einsetzen des Femurteils 3 und des Inlays 31 ohne die Notwendigkeit einer Überdehnung der Kniebandstrukturen oder gar eine Ablösung derselben.

Indem der axiale Teil der Ausnehmung 12 im Tibiateil 2 etwas tiefer gebohrt ist als der divergierende Teil 46, endet das Einsetzen des Femurteils 3 und des Inlays 31 mit einer axial gerichteten Absenkung des Inlays in das Tibiaplateau 6, wo ein vollendeter Formschluß des Inlays 31 mit dem Seitenbord 33 der Tragfläche 36 des Tibiaplateaus 6 erzielt wird. Indem der Hülsenkörper 35 des Inlays 31 im wesentlichen komplementär zur Innenform der divergierenden Ausnehmung 12 des Tibiateils 2 ausgebildet ist, endet das Einsetzen ferner mit einem vollendeten Formschluß des Hülsenkörpers 35 mit der Ausnehmung 12.

Fig. 3 zeigt den Endzustand nach dem Einsetzen. Anhand dieser Darstellung ist erkennbar, daß die schräge anteriore Innenkante 43 der Ausnehmung 12 mit einer Tangente 44 an einen Kreisbogen 45 um die Schwenkachse 29 des Gelenkkopfes 18 zusammenfällt, welche unter einem Winkel α zur Tibiaachse 20 geneigt ist.

Fig. 4 zeigt eine schematische Draufsicht auf das Tibiaplateau 6 und verdeutlicht die Formgebung der Ausnehmung 12. Diese besteht nämlich aus einem axialen Teil 40 und einem divergierenden Teil 46, wobei sich der divergierende Teil 46 von dem axialen Teil 40 in anteriorer Richtung (in der Darstellung oben) erstreckt. In der dargestellten Ausführungsform hat die Ausnehmung 12 in der Tiefe ihrer Erstreckung im Tibiaschaft 4 eine im wesentlichen kreissymmetrische Querschnittsform, die sich in Richtung auf das Tibiaplateau 6 zu der Querschnittsform eines Langlochs 41 entwickelt. Selbstverständlich sind auch andere Querschnittsformen des unteren Teils der Ausnehmung 12 denkbar, z.B. quadratische, mehreckige, ovale oder rechteckige. Gegebenenfalls kann es vorteilhaft sein, die Seitenwände 47, 48 der Ausnehmung 12 ebenfalls von unten nach oben divergierend auszubilden, wie es in Fig. 4 gestrichelt dargestellt ist, und zwar sowohl nach anterior als auch zum Tibiaplateau hin, so daß eine trapezähnliche Querschnittsform der Ausnehmung 12 entsteht.

Das mit der beschriebenen Kniegelenkendoprothese erzielbare schonende Zusammenkoppeln der Prothesenteile sieht vor, daß zunächst das Inlay 31 durch Aufstecken auf den Kupplungszapfen 16 mit dem Femurteil 3 verbunden wird, und danach diese beiden Bauteile durch den in Fig. 2 dargestellten schrägen Ansatz in das Tibiateil 2 eingesetzt werden.

## Patentansprüche

1. Kniegelenkendoprothese, mit
einem Femurteil (1), das am unteren Ende eines Femurschaftes (3) konvex gekrümmte Kondylenschalen (5, 7) mit ersten Gleitflächen (9, 11) aufweist, die dorsal zwei voneinander beabstandete, einen interkondylären Zwischenraum bildende Seitenwände (13, 15) besitzen;
einem Tibiateil (2), das am oberen Ende eines Tibiaschaftes (4) ein Tibiaplateau (6) aufweist, auf dem den ersten Gleitflächen (9, 11) der Kondylenschalen (5, 7) entsprechende zweite Gleitflächen (8, 10) ausgebildet sind, und das eine sich axial in dem Tibiaschaft (4) erstreckende Ausnehmung (12) aufweist; und mit
einem Kupplungsteil (14), das am oberen Ende eines drehbar in der Ausnehmung (12) aufgenommenen Kupplungszapfens (16) einen in den interkondylären Zwischenraum hineinragenden Gelenkkopf (18) aufweist, der um eine quer zur Femurachse (21) verlaufende Schwenkachse (29) schwenkbar gelagert ist,
**dadurch gekennzeichnet, daß**
sich der Querschnitt der Ausnehmung (12) des Tibiateils (2) zum Tibiaplateau (6) hin von der Tiefe der Ausnehmung einseitig zu einem Langloch (41) im Tibiaplateau (6) vergrößert.

2. Kniegelenkendoprothese nach Anspruch 1,
**dadurch gekennzeichnet, daß**
die Längsachse (42) des Langlochs (41) im wesentlichen von posterior nach anterior verläuft.

3. Kniegelenkendoprothese nach Anspruch 1 oder 2,
**dadurch gekennzeichnet, daß**
die anteriore, von der Tiefe der Ausnehmung schräg in Richtung des Tibiaplateaus (6) verlaufende Innenkante (43) der divergierenden Ausnehmung (12) bei zusammengesetzter Prothese eine Tangente (44) an einen Kreisbogen (45) um die Schwenkachse (29) des Gelenkkopfes (18) ist.

4. Kniegelenkendoprothese nach einem der Ansprüche 1 bis 3,
**dadurch gekennzeichnet, daß**
die schräge Innenkante (43) der divergierenden Ausnehmung (12) wahlweise geradlinig, konkav oder konvex gewölbt, oder in Form einer Kombination von mindestens zwei dieser Formen ausgebildet ist.

5. Kniegelenkendoprothese nach einem der Ansprüche 1 bis 4,
**dadurch gekennzeichnet, daß**
die Seitenwände (47, 48) der divergierenden Ausnehmung (12) nach anterior und/oder zum Tibiaplateau (6) hin divergieren.

6. Kniegelenkendoprothese nach einem der vorstehenden Ansprüche,
**dadurch gekennzeichnet, daß**
der axiale Teil der Ausnehmung (12) eine größere axiale Erstreckung aufweist als der divergierende Teil.

7. Kniegelenkendoprothese nach Anspruch 6,
**dadurch gekennzeichnet, daß**
die zusätzliche axiale Erstreckung wenigstens die Höhe eines die Tragfläche (36) des Tibiaplateaus (6) begrenzenden Seitenbords (33) beträgt.

8. Kniegelenkendoprothese nach einem der vorstehenden Ansprüche, bei dem zwischen der Tragfläche (36) und den ersten Gleitflächen (9, 11) der Kondylenschalen (5, 7) ein Inlay (31) mit einer Bodenfläche (34), zweiten Gleitflächen (8, 10) auf der Oberseite und einer Bohrung (32) zum Einführen des Kupplungszapfens (16) angeordnet ist,
**dadurch gekennzeichnet, daß**
an der Bodenfläche (34) ein Hülsenkörper (35) angesetzt ist, dessen Hohlraum mit der Bohrung (32) fluchtet.

9. Kniegelenkendoprothese nach Anspruch 8,
**dadurch gekennzeichnet, daß**
der Hülsenkörper (35) im wesentlichen zur Innenform der Ausnehmung (12) des Tibiateils (2) komplementär ausgebildet ist.

10. Kniegelenkendoprothese nach Anspruch 8 oder 9,
**dadurch gekennzeichnet, daß**
der Hülsenkörper (35) als separates Bauteil ausgebildet und im Inlay (31) eine Aussparung zum Einsetzen des Hülsenkörpers (35) vorgesehen ist.

11. Kniegelenkendoprothese nach Anspruch 10,
**dadurch gekennzeichnet, daß**
die Oberkante (51) des Hülsenkörpers (35) nach dem Einsetzen in die Aussparung in dem Inlay (31) mit der Oberseite (52) des Inlays (31) fluchtet.

12. Kniegelenkendoprothese nach Anspruch 8 oder 9, **dadurch gekennzeichnet, daß**
der Hülsenkörper (35) als separates Bauteil ausgebildet ist und die Oberkante (51) des Hülsenkörpers (35) nach dem Einsetzen in die Ausnehmung (12) des Tibiateils (2) mit der Tragfläche (36) des Tibiaplateaus (6) fluchtet.

13. Kniegelenkendoprothese nach einem der Ansprüche 8 bis 12,
**dadurch gekennzeichnet, daß**
der Hülsenkörper (35) längs der Tibiaachse (20) geteilt ausgebildet ist, so daß eine posteriore und eine anteriore Halbschale entsteht.

## Claims

1. A knee-joint endoprosthesis, comprising a femural component (1) having convex condylar shells (5, 7) with first sliding surfaces (9, 11) at the lower end of a femural shaft (3), the sliding surfaces (9, 11) being provided dorsally with two spaced side walls (13, 15) forming an intercondylar space, comprising a tibial component (2) having a tibial plate (6) at the upper end of a tibial shaft (4), two sliding surfaces (8, 10) corresponding to the first sliding surfaces (9, 11) of the condylar shells (5, 7) being formed on the tibial plate (6), which is provided with a recess (12) extending axially into the tibial shaft (4), and comprising a connecting component (14) which, at the upper end of a connecting pin (16) rotatably mounted in the recess (12), has an articulation head (18) projecting into the intercondylar space and mounted so as to be pivotable about a pivoting axis (29) extending transversely to the femural axis (21), **characterised in that** the cross-section of the recess (12) in the tibial component (2) increases on one side from the bottom of the recess in the direction of the tibial plate (6) to form a slot (41) therein.

2. A knee-joint endoprosthesis according to claim 1, **characterised in that** the longitudinal axis (42) of the slot (41) extends substantially from back to front.

3. A knee-joint endoprosthesis according to claim 1 or 2, **characterised in that** the anterior inner edge (43) of the diverging recess (12), extending from the bottom of the recess obliquely towards the tibial plate (6), is a tangent (44) to an arc (45) around the pivoting axis (29) of the articulation head (18) when the prosthesis is assembled.

4. A knee-joint endoprosthesis according to any one of claims 1 to 3, **characterised in that** the oblique inner edge (43) of the diverging recess (12) is optionally straight, concave or convex, or is formed as a combination of at least two of these forms.

5. A knee-joint endoprosthesis according to any one of claims 1 to 4, **characterised in that** the side walls (47, 48) of the diverging recess (12) diverge towards the front and/or towards the tibial plate (6).

6. A knee-joint endoprosthesis according to any one of the preceding claims, **characterised in that** the axial part of the recess (12) has a greater axial extent that the diverging part.

7. A knee-joint endoprosthesis according to claim 6, **characterised in that** the additional axial extent is at least the height of a lateral edge (33) forming the boundary of the supporting surface (36) of the tibial plate (6).

8. A knee-joint endoprosthesis according to any one of the preceding claims, wherein an inlay (31), comprising a base surface (34), two sliding surfaces (8, 10) on the upper side and a bore (32) for insertion of the connecting pin (16), is arranged between the supporting surface (36) and the first sliding surfaces (9, 11) of the condylar shells (5, 7), **characterised in that** a socket piece (35), the cavity of which is in alignment with the bore (32), is attached to the base surface (34).

9. A knee-joint endoprosthesis according to claim 8, **characterised in that** the socket piece (35) is substantially complementary to the inner shape of the recess (12) in the tibial component (2).

10. A knee-joint endoprosthesis according to claim 8 or 9, **characterised in that** the socket piece (35) is formed as a separate component, and a recess for insertion of the socket piece (35) is provided in the inlay (31).

11. A knee-joint endoprosthesis according to claim 10, **characterised in that** the upper edge (51) of the socket piece (35) is aligned with the upper side (52) of the inlay (31) after insertion into the recess.

12. A knee-joint endoprosthesis according to claim 8 or 9, **characterised in that** the socket piece (35) is formed as a separate component, and the upper edge (51) of the socket piece (35) is aligned with the supporting surface (36) of the tibial plate (6) after insertion into the recess (12).

13. A knee-joint endoprosthesis according to any one of claims 8 to 12, **characterised in that** the socket piece (35) is divided along the tibial axis (20) to form a posterior and an anterior half-shell.

## Revendications

1. Endoprothèse de l'articulation du genou, comprenant
un fémur artificiel (1), dont la tige de fémur (3) porte sur son extrémité inférieure des coques condyliennes (5, 7) à courbure convexe et munies de premières surfaces de glissement (9, 11), qui possèdent, dans la partie dorsale, deux parois latérales (13, 15) disposées à une distance donnée l'une de l'autre et formant un intervalle intercondylien ;
un tibia artificiel (2), dont la tige de tibia (4) est munie sur son extrémité supérieure d'un plateau tibial (6), sur lequel sont formées les deuxièmes surfaces de glissement (8, 10) correspondant aux premières surfaces de glissement (9, 11) des coques condyliennes (5, 7), et qui comporte une cavité (12) qui s'étend dans le sens axial à l'intérieur de la tige de tibia (4) ; et
une pièce de couplage (14), qui comporte, sur l'extrémité supérieure d'une tige de couplage (16) logée de manière à pouvoir tourner dans la cavité (12), une tête d'articulation (18) qui s'engage dans l'intervalle intercondylien et qui est logée de manière à pouvoir pivoter autour d'un axe de pivotement (29) disposé transversalement à l'axe du fémur (21),
**caractérisée en ce que**
la section de la cavité (12) dans le tibia artificiel (2) s'évase sur un côté à partir du fond de la cavité en remontant vers le plateau tibial (6) pour former un trou oblong (41) dans le plateau tibial (6).

2. Endoprothèse de l'articulation du genou selon la revendication 1, **caractérisée en ce que** l'axe longitudinal (42) du trou oblong (41) s'étend sensiblement de la partie postérieure vers la partie antérieure.

3. Endoprothèse de l'articulation du genou selon la revendication 1 ou 2, **caractérisée en ce que** le bord intérieur (43) antérieur de la cavité (12) divergente, qui s'étend en oblique depuis le fond de la cavité vers le plateau tibial (6), forme dans la prothèse en position assemblée une tangente (44) d'un arc de cercle (45) tracé autour de l'axe de pivotement (29) de la tête d'articulation (19).

4. Endoprothèse de l'articulation du genou selon l'une quelconque des revendications 1 à 3, **caractérisée en ce que** le bord intérieur (43) oblique de la cavité (12) divergente peut être conçu au choix sous forme rectiligne, avec une courbure concave ou convexe ou sous forme de combinaison d'au moins deux de ces formes.

5. Endoprothèse de l'articulation du genou selon l'une quelconque des revendications 1 à 4, **caractérisée en ce que** les parois latérales (47, 48) de la cavité divergente (12) divergent vers la partie antérieure et/ou vers le plateau tibial (6).

6. Endoprothèse de l'articulation du genou selon l'une quelconque des revendications précédentes, **caractérisée en ce que** la partie axiale de la cavité (12) présente une dimension axiale supérieure à la partie divergente.

7. Endoprothèse de l'articulation du genou selon la revendication 6, **caractérisée en ce que** la dimension axiale supplémentaire est au moins égale à la hauteur d'un bord latéral (33) délimitant la surface de support (36) du plateau tibial (6).

8. Endoprothèse de l'articulation du genou selon l'une quelconque des revendications précédentes, dans laquelle, entre la surface de support (36) et les premières surfaces de glissement (9, 11) des coques condyliennes (5, 7), est disposé un insert (31) comportant un fond (34), deux surfaces de glissement (8, 10) sur la face supérieure et une forure (32) destinée à recevoir la tige de couplage (16), **caractérisée en ce qu'**un manchon (35), dont la cavité est alignée avec la forure (32), est monté contre le fond (34).

9. Endoprothèse de l'articulation du genou selon la revendication 8, **caractérisée en ce que** le manchon (35) est conçu avec une forme sensiblement complémentaire à la forme intérieure de la cavité (12) du tibia artificiel (2).

10. Endoprothèse de l'articulation du genou selon la revendication 8 ou 9, **caractérisée en ce que** le manchon (35) est conçu sous forme de pièce séparée et un évidement destiné à recevoir le manchon (35) est prévu dans l'insert (31).

11. Endoprothèse de l'articulation du genou selon la revendication 10, **caractérisée en ce que** le bord supérieur (51) du manchon (35), après que celui-ci est inséré dans l'évidement réalisé dans l'insert (31), forme un assemblage affleuré avec la face supérieure (52) de l'insert (31).

12. Endoprothèse de l'articulation du genou selon la revendication 8 ou 9, **caractérisée en ce que** le manchon (35) est conçu sous forme de pièce séparée et le bord supérieur (51) du manchon (35), après que celui-ci est inséré dans la cavité (12) réalisée dans le tibia artificiel (2), forme un assemblage affleuré avec la surface de support (36) du plateau tibial (6).

13. Endoprothèse de l'articulation du genou selon l'une quelconque des revendications 8 à 12, **caractérisée en ce que** le manchon (35) est séparé le long de l'axe du tibial (20) de manière à former une demi-coque postérieure et une demi-coque antérieure.
